(19) Europäisches Patentamt
European Patent Office
Office européen des brevets

(11) EP 2 551 351 A1

(12) **EUROPEAN PATENT APPLICATION**

(43) Date of publication:
**30.01.2013 Bulletin 2013/05**

(51) Int Cl.:
***C12P 17/10*** *(2006.01)*

(21) Application number: **11175965.0**

(22) Date of filing: **29.07.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Interquim, S.A.**
**08173 Sant Cugat del Vallés, Barcelona (ES)**

(72) Inventors:
- **Marquillas Olondriz, Francisco**
  **08017 Barcelona (ES)**
- **Planas Sauter, Antoni**
  **08198 Sant Cugat Del Valles (ES)**
- **Perez Javierre, Xavier**
  **08230 Matadepera (ES)**

(74) Representative: **ZBM Patents**
**Zea, Barlocci & Markvardsen**
**Plaza Catalunya, 1**
**08002 Barcelona (ES)**

# (54) Process for production of optically active (R)-(-)-1-(2,4-dichloro-phenyl)-2-imidazole-1-yl-ethanol

(57) A process for the manufacture of (*R*)-(-)-1-(2,4-dichloro-phenyl)-2-imidazole-1-yl-ethanol (**I**) by biocatalytic asymmetric reduction of 1-(2,4-dichloro-phenyl)-2-imidazole-1-yl-ethanone (**II**) is provided. The invention also provides a process for the manufacture of Arasertaconazole from (**I**).

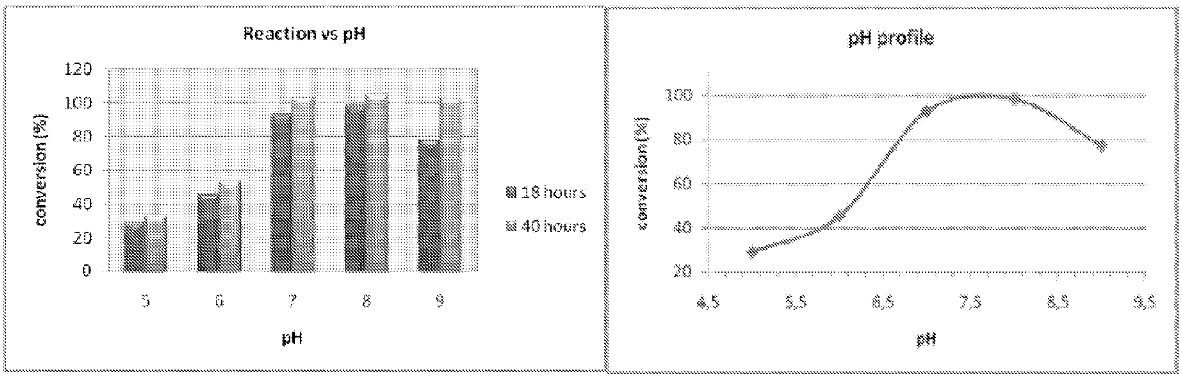


*FIGURE 1*

EP 2 551 351 A1

## Description

The present invention provides a process for producing optically active (*R*)-(-)-1-(2,4-dichloro-phenyl)-2-imidazole-1-yl-ethanol ((*R*)-**DCPI**) from 1-(2,4-dichloro-phenyl)-2-imidazole-1-yl-ethanone (**DCPE**) using an asymmetric reduction by a microorganism with commercial advantages in simple and easy manner.

## BACKGROUND OF THE INVENTION

(*R*)-(-)-1-(2,4-dichloro-phenyl)-2-imidazole-1-yl-ethanol (**I**) is a chiral intermediate required for the synthesis of a compound known as (*R*)-Sertaconazole and its salts, which is an antifungal agent with activity against fungi and yeast in humans and animals.

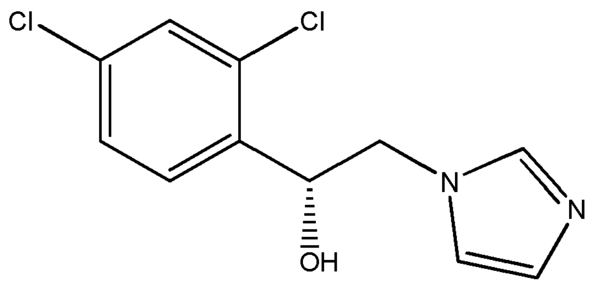

**(*R*)-DCPI** (**I**)

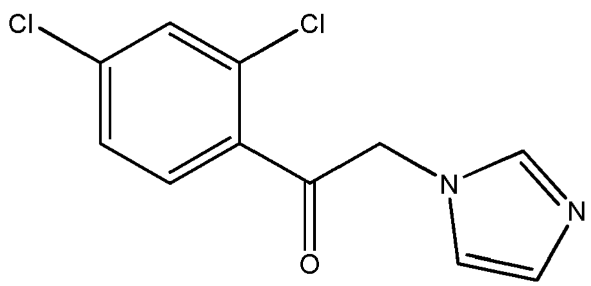

**DCPE** (**II**)

For the production of the optically active intermediate ***(R)*-DCPI** there are known processes such as:

- ➢ Resolution of the corresponding racemic mixture with an optically active acid according to the method described in GB patent No. 1244530 and in Lämmerhofer M. and Linder W. (Chirality, 1994, 6, 261 - 269);
- ➢ Enantioselective reduction of 1-(2,4-dichloro-phenyl)-2-imidazole-1-yl-ethanone (**II**) using (-)-β-chlorodiisopinocamphenyl borane with an acceptable optical purity (WO03/068770);
- ➢ Biosynthetic resolution of the racemic alcohol with *Alcaligene sp.* lipase or *Pseudomonas stutzeri* lipase (CN1765887); and
- ➢ The bioreduction of 2-chloro-1-(2,4-dichlorophenyl)ethanone with ADH-A (alcohol dehydrogenase A) in the presence of NADH (1,4-dihydronicotinamide adenine dinucleotide) (Mangas-Sanchez, J. et al., Journal of Organic Chemistry, 2011, 76(7), 2115-2122) provides (R)-2-chloro-1-(2,4-dichlorophenyl)ethanol, a related chiral building block, which can be chemically transformed into ***(R)*-DCPI** by nucleophilic substitution with imidazole using sodium hydride (NaH) and N,N-dimethylformamide (DMF) as solvent at 100°C. The authors reported that the intended reduction of **DCPE** to obtain an enantiopure **DCPI** by means of an isolated alcohol dehydrogenase failed. This brought the authors to discover the above alternative biosynthetic route to produce ***(R)*-DCPI**.

The method here reported is the first green biocatalytic process for the production of (***R***)-**DCPI** intermediate with high optical purity from **DCPE**. In addition, there has been no known process for the production of optically active

(*R*)-(-)-1-(2,4-dichloro-phenyl)-2-imidazole-1-yl-ethanol (**I**) from 1-(2,4-dichloro-phenyl)-2-imidazole-1-yl-ethanone (**II**) by using a microorganism capable of asymmetrically reducing the 1-(2,4-dichloro-phenyl)-2-imidazole-1-yl-ethanone (**II**).

## SUMMARY OF THE INVENTION

The present invention provides a process for producing optically active (***R***)-**DCPI** (**I**) in a simple, efficient and easy manner by biocatalytic asymmetric reduction of the ketone compound **DCPE** (**II**) with a microorganism.

Thus, it is an aspect of the invention to provide a biocatalytic method of reducing 1-(2,4-dichloro-phenyl)-2-imidazole-1-yl-ethanone (**II**) to the optically active (*R*)-(-)-1-(2,4-dichloro-phenyl)-2-imidazole-1-yl-ethanol (**I**).

More particularly, the process for producing optically active **(*R*)-DCPI** (I) comprises permitting a microorganism to act on 1-(2,4-dichloro-phenyl)-2-imidazole-1-yl-ethanone (**II**) said microorganism having ketone asymmetric reduction activity; and recovering or harvesting the optically active (*R*)-(-)-1-(2,4-dichloro-phenyl)-2-imidazole-1-yl-ethanol (**I**) produced.

The present invention also relates to a process for the manufacture of Arasertaconazole from **(*R*)-DCPI** obtained as described above.

## BRIEF DESCRIPTION OF THE FIGURES

**Figure 1**: Biocatalytic reduction of DCPE by *Rhodococcus ruber.* Percentage of conversion of DCPE at different pH (pH = 5 to pH = 9) and at 18 and 40 hours reaction (left panel) and pH profile (right panel).

## DETAILED DESCRIPTION OF THE INVENTION

The authors of the present invention have found that a microorganism having ketone asymmetric reduction activity is capable to act on 1-(2,4-dichlorophenyl)-2-imidazole-1-yl-ethanone (**DCPE**) to produce the corresponding optically active (*R*)-(-)-1-(2,4-dichloro-phenyl)-2-imidazole-1-yl-ethanol ((*R*)-DCPI) in a very easy, simple, and effective manner.

Therefore, in a first aspect, the present invention provides a process for producing optically active (*R*)-(-)-1-(2,4-dichloro-phenyl)-2-imidazole-1-yl-ethanol (**I**) which comprises bringing into contact a compound of formula (**II**) with a microorganism, wherein said microorganism has ketone asymmetric reduction activity; and recovering the compound of formula **(I)** so produced.

The microorganism employed in this process may be any microorganism capable of producing (*R*)-DCPI from DCPE. Thus, as used herein, the term "microorganism" refers to any microscopic organism, including bacteria, fungi, microalgae, protists, and archaea. Additionally, in order to perform the method of the present invention, said microorganism can be in the form of individual cells, a culture of said cells, or processed cells of said microorganism.

As used herein, the term "processed cells" include treated cells such as, for example, dried cells or lyophilized cells. Thus, the microorganisms according to the present invention can be employed as a wet agglomerate of cells of viable or dead organisms. In a preferred embodiment, microorganisms are employed as a washed agglomerate of cells which has been isolated by centrifugation and taken up in physiological saline.

Any microorganism which has the ability to form the optically active (*R*)-(-)-1-(2,4-dichloro-phenyl)-2-imidazole-1-yl-ethanol (I) from 1-(2,4-dichloro-phenyl)-2-imidazole-1-yl-ethanone (II) can be employed in the method of the present invention. Illustrative, non limitative examples of microorganisms having such activity are those belonging to the genus *Saccharomyces, Schizochytrium, Lactobacillus, Rhodococcus* and *Pseudomonas*. In particular, examples of said microorganisms are those belonging to the species *Saccharomyces cerevisiae, Schizochytrium limacinum, Lactobacillus kefiri, Rhodococcus ruber* and *Pseudomonas fluorescens. Rhodococcus ruber* is the preferred microorganism according to the invention.

In a particular embodiment, specific examples of the strains capable of producing **(*R*)-DCPI** from **DCPE** may include *Saccharomyces cerevisiae* CECT 1170 strain, *Lactobacillus kefiri* DSMZ 20587 strain, *Rhodococcus ruber* DSMZ 44541 strain, *Pseudomonas fluorescens* CECT 378 strain, and *Schizochytrium limacinum* strain ATCC MYA-1381.

The microorganisms identified by CECT numbers are listed in the catalogue from the "Colección Española de Cultivos Tipo" and are available from the CECT webpage (www.cect.org). The microorganisms identified by DSMZ numbers are listed in the catalogue of strains published by the "Deutsche Sammlung von Mikroorganismen und Zellkulturen" and are available from the DSMZ webpage (www.dsmz.de). The microorganisms identified by ATCC numbers are listed in the catalogue from the "American Type Culture Collection" and are available from the ATCC webpage (www.atcc.org).

In the present invention, the microorganisms can be wild-type strains, variants or recombinants thereof obtained through genetic engineering techniques such as cell fusion or gene manipulation as far as those cells have the capacity to form the optically active (*R*)-(-)-1-(2,4-dichloro-phenyl)-2-imidazole-1-yl-ethanol (I) from 1-(2,4-dichloro-phenyl)-2-imidazole-1-yl-ethanone (II).

The medium for growing microorganisms for use in the present invention is not restricted as far as they may grow and multiply therein. The medium normally includes, for example, carbon sources (saccharides; alcohols; organic acids; hydrocarbons; other carbon sources) and nitrogen sources (inorganic acid ammonium salts; organic acid ammonium salts; inorganic or organic nitrogen containing compounds such as yeast extract, meat extract, malt extract, soy extract, peptone, polypeptone, urea, etc.). In the medium, there may be incorporated appropriate amounts of those nutrients which are commonly employed in the cultivation of microorganisms, such as inorganic salts, trace metal salts and vitamins. Where necessary, factors that promote multiplication of a microorganism, factors which improve its ability to produce the object compound of the invention and compounds maintaining the pH of the medium (such as buffer substances) may be added. In a particular case, the preferred media for growing yeast cells, bacterial cells and microalgae are as follows:

Medium for yeast:

| | |
|---|---|
| D(+) glucose | 20 g/l |
| Meat peptone | 5.0 g/l |
| Casein peptone | 5.0 g/l |
| pH | 5.6 |

Medium for bacterium:

| | |
|---|---|
| Tryptone | 15 g/l |
| Soya peptone | 5.0 g/l |
| Sodium chloride | 5.0 g/l |
| Potassium phosphate | 2.5 g/l |
| pH | 7.2 |

Medium for microalgae:

| | |
|---|---|
| Yeast extract | 1.0 g |
| Peptone | 1.0 g |
| D(+) glucose | 5.0 g |
| Seawater | 1.0 L |

The cultivation of the microorganism may be carried out under optimal conditions for the growth and multiplication of the microorganisms, for example at a pH of 5 - 9 and a temperature in the range of 20 - 50°C. The cultivation may be performed in aerobic or anaerobic conditions for one to five days.

In a first step of the process of the invention, a compound of formula (II) is brought into contact with a microorganism according to the present invention. There are well known techniques which can be employed according to the process of the invention in order to put into contact the compound of formula (II) with the microorganism and allow it to reduce the **DCPE** asymmetrically.

As an example, said process comprises mixing or blending the **DCPE** compound with a culture broth in order to conduct the reaction.

Alternatively, the microbial cells are separated from the culture broth (for example by centrifugation, filtration, etc) resuspended, either as they are or after washing, in a buffer solution, water or the like, and then the **DCPE** is added to the resultant cell suspension (resting cells) in order to conduct the reaction.

In another embodiment, not viable cells but a treated preparation of cells (such as dried cells or lyophilized preparations of cells) can be used according to the method of the present invention.

In another particular embodiment, whole cells or cell preparations as described above can be immobilized by known methods for use in the process of the present invention. As an example, the microorganisms can be physically inserted in a polymer matrix. A very wide variety of materials is used for the preparation of matrices of this type, using many different procedures, for example using polymers of unsaturated carboxylic acids with low molecular weight, such

as acrylic acid and its derivatives.

In many cases, a source of reducing power as NAD(P)H, glucose, sucrose, ethanol, isopropanol, methanol, salt of formate and the like are added to achieve better yields. **DCPE** (**II**) may be used as it is, or solubilized in an organic solvent that does not interfere with the reaction (for example alcohols, dimethyl sulfoxide (DMSO)).

The reaction conditions are set in the margin where the activity of the microorganism or the preparation thereof is optimal. Thus, in a preferred embodiment, such conditions include a pH range of 5 - 9, a temperature of 20 - 50°C, with stirring for about 1 to 120 hours. In another preferred embodiment, the cell concentration is in the range of 1 - 300 g/l, on a wet cell basis.

The optically active **(*R*)-DCPI** (**I**) produced according to the method of the invention can be recovered or harvested by conventional procedures such as, for example, separation of cells or cell preparations thereof (centrifugation, filtration) and purification (extraction with organic solvent, crystallization, recrystallization, chromatography, concentration and distillation). Any of the organic solvents (alcohols such as butanol; hydrocarbons such as hexane, cyclohexane, toluene; esters such as ethyl acetate; ketones; ethers and mixtures thereof) may be employed. Halogenated hydrocarbons such as chloroform and methylene chloride can be used.

As mentioned above, the (*R*)-(-)-1-(2,4-dichloro-phenyl)-2-imidazole-1-yl-ethanol (**I**), obtained according to the process of the present invention can be used in the preparation of the (*R*)-enantiomer of Sertaconazole (Arasertaconazole)

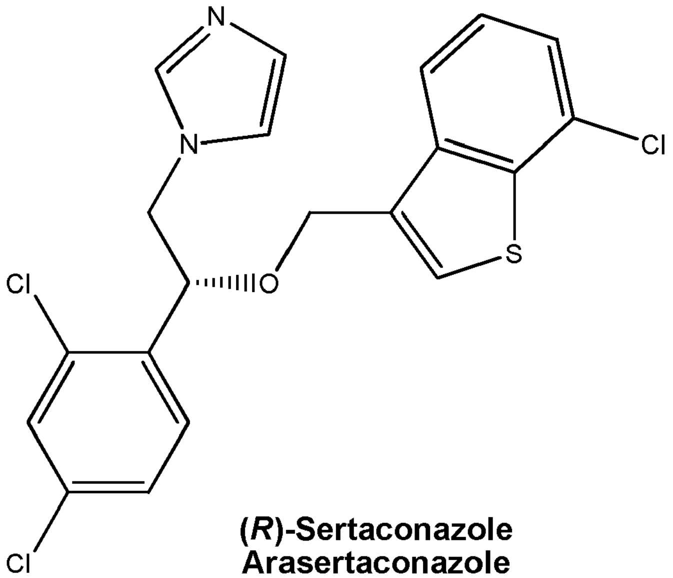

**(*R*)-Sertaconazole**
**Arasertaconazole**

Accordingly, in a second aspect, the present invention also refers to a process for the manufacture of the (*R*)-enantiomer of Sertaconazole (Arasertaconazole) which comprises bringing into contact a compound of formula (**II**) with a microorganism, wherein said microorganism has ketone asymmetric reduction activity; and O-alkylating the resulting compound **(*R*)-DCPI** (I) with a compound of formula (**III**)

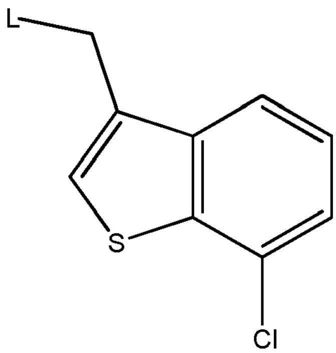

(**III**)

wherein L is a leaving group.

Common leaving groups include substituted benzoates, halides, sulfonates, and perfluoroalkane sulfonates. Non-limitative examples of leaving groups comprise chloride, bromide, iodide, tosylate, mesyl, and trifluoromethylsulfonyl.
Further treatment of Arasertaconazole with appropriate acids may form pharmaceutically acceptable salts. Accordingly, the arasertaconazole thus obtained, or a salt pharmaceutically acceptable thereof, can be used in the preparation of pharmaceutical compositions.
The invention will now be described in more detail, by way of examples which in no way are meant to limit the scope of the invention, but, rather, these examples will serve to illustrate the invention with reference to the accompanying figures.

## EXAMPLES

An example of a general procedure for the method of the present invention is explained below:
The process can be divided into three steps. The first one includes the microorganism growth and the isolation of the biomass in order to obtain viable cells or a preparation thereof. Second step corresponds to the biotransformation process carried out by the microorganism or the preparation thereof obtained in the first step. Finally, an analytical procedure is performed in order to determine the yield and the enantiomeric purity of the **(*R*)-DCPI** obtained.

*1. Cell growth*

In order to culture the microorganisms used in this invention, any medium may be used without restriction, as long as the microorganisms can grow therein. Nevertheless, typical preferred media are as follows:
Medium for yeast:

| | |
|---|---|
| D(+) glucose | 20 g/l |
| Meat peptone | 5.0 g/l |
| Casein peptone | 5.0 g/l |
| pH | 5.6 |

Medium for bacterium:

| | |
|---|---|
| Tryptone | 15 g/l |
| Soya peptone | 5.0 g/l |
| Sodium chloride | 5.0 g/l |
| Potassium phosphate | 2.5 g/l |
| pH | 7.2 |

Medium for microalgae:

| | |
|---|---|
| Yeast extract | 1.0 g |
| Peptone | 1.0 g |
| D(+) glucose | 5.0 g |
| Seawater | 1.0 L |

A 2 L Erlenmeyer flask charged with 500 ml of the cell-preparing medium described above, was autoclave-sterilized at a temperature of 121°C for 15 minutes, and inoculated with one of the microorganisms described before. The inoculated flask was incubated under shaking (150 - 220 rpm) at the optimal growing temperature (normally 30°C) for two days. When the growing culture achieved an $OD_{600}$ of 8 or above, cells were isolated by centrifugation. Subsequently, cells were washed with sodium chloride 0.9% and resuspended in reaction buffer (100 mM sodium phosphate buffer pH 8 at 30°C) to obtain resting cells.

*2. General biotransformation conditions*

A reaction flask with 100 mg of **DCPE** (**II**) and 160 mg of glucose suspended in 100 mM sodium phosphate buffer was charged with a volume of resting cells prepared above to achieve the desired cell concentration in a final volume of 40 ml. The reaction was conducted on an orbital shaker (200 rpm) at 30°C for three days.

After completion of the reaction, the pH of the suspension was shift to a pH of 12 and the product was extracted with ethyl acetate (3x20 ml). Separation between phases was achieved by centrifugation (10000 rpm, 15 min). Solvent was evaporated to achieve a yellow - brown solid, which was dissolved in 1M hydrochloric acid solution. Impurities were extracted with ethyl acetate (3x10 ml). Then, solution was adjusted to pH > 12 with sodium hydroxide and ***(R)*-DCPI** (**I**) was extracted with ethyl acetate (3x10 ml). Solvent was evaporated and **(*R*)-DCPI** (**I**) produced and purified was dissolved in an appropriate solvent for high performance liquid chromatography (HPLC) analysis.

*3. Analytical procedure for the determination of yields and enantiomeric purity*

Yield and enantiomeric purity determination for the bioconversion of **DCPE** into ***(R)*-DCPI** was carried out by HPLC using a chiral resolution column [Chiradex (5 μm, 250 x 4.0 mm); mobile phase (methanol/triethylamine 0.2% pH 6 = 25/75); column temperature: 30°C; detection: absorbance 225 nm; flow rate: 0.8 ml/min]. Retention time aprox.: **DCPE** 11 min; **(*R*)-DCPI** 17 min; ***(S)*-DCPI** 19 min.

Within this general procedure, different parameters can be further adjusted or optimized for each particular case (such as microorganisms, co-solvents, and co-substrates). Those are:

- **DCPE**-Biomass ratio: the ratio between starting material **DCPE** and biomass (wet cell basis);
- **DCPE**-Biomass ratio in the presence of 10% DMSO;
- Co-substrate effect: influence of the co-substrate used as reducing power;
- Co-solvent effect: influence of the co-solvent used to solubilize the **DCPE;**
- Temperature set-up study;
- Reaction time set-up study;
- Reaction pH set-up study;

The study has been made using *Rhodococcus ruber* resting cells.

**EXAMPLE 1: DCPE-Biomass ratio**

In order to study the relationship between the amount of the compound **DCPE** and cell concentration (biomass), resting cells of *Rhodococcus ruber* were prepared as described in the general procedure. Several reactions were performed at different **DCPE**-resting cells ratio and the yield and enantiomeric purity of the product obtained **(*R*)-DCPI** (**I**) were determined (Table 1).

A reaction flask with 100 mg of **DCPE** (**II**) and 160 mg of glucose suspended in 100 mM sodium phosphate buffer was charged with a volume of *Rhodococcus ruber* resting cells prepared as described above to achieve the desired cell concentration in a final volume of 40 ml. The reaction was conducted on an orbital shaker (200 rpm) at 30°C for three days. For work-up operations, general procedure was followed. The results are shown in Table 1.

Table 1

| ***DCPE-Biomass ratio* (*mg DCPE/g cells*)** | ***Conversion* (%)** | ***Yield* (%)** | ***Enantiomeric Purity* (*% R*)** |
|---|---|---|---|
| 28 | 93 | 84 | > 99 |
| 56 | 87 | 64 | > 99 |
| 112 | 39 | 35 | > 99 |
| 1124 | 4 | 3 | > 99 |

For a DCPE-biomass ratio below 50 mg/g, high conversions were achieved. Yields after work-up exceed 60%, with high enantiomeric purity for the **(*R*)-DCPI.**

**EXAMPLE 2: DCPE-Biomass ratio in the presence of 10% DMSO**

In order to study the influence of 10% DMSO presence in the **DCPE**-biomass ratio established in the first

example. For that purpose, resting cells of *Rhodococcus ruber* were prepared as described in the general procedure and several reactions were performed at different **DCPE**-resting cells ratio. The yield and enantiomeric purity of the product obtained **(R)-DCPI (I)** were determined (Table 2).

A reaction flask with 100 mg of **DCPE** dissolved in 4 ml DMSO and 160 mg of glucose is charged with 100 mM sodium phosphate buffer and a volume of resting cells prepared above to attain the desired cell concentration in a final volume of 40 ml. The reaction was conducted on an orbital shaker (200 rpm) at 30°C for three days.

After completion of the reaction, the pH of the suspension was shift to a pH of 12 and product was extracted with ethyl acetate (3x20 ml). Separation between phases is achieved by centrifugation (10000 rpm, 15 min). Solvent was evaporated to achieve yellow - brown oil. DMSO traces were removed by co-distillation with water in a rotary evaporator. The next steps followed the general procedure described above. The results are shown in Table 2.

Table 2

| ***DCPE-Biomass ratio (mg DCPE/g cells)*** | ***Conversion* (%)** | ***Yield* (%)** | ***Enantiomeric Purity (% R)*** |
|---|---|---|---|
| 28 | 100 | 80 | > 99 |
| 56 | 78 | 66 | > 99 |
| 112 | 36 | 33 | > 99 |
| 1124 | 2 | 1 | > 99 |

Even though in some cases conversion values were higher than in Example 1, the yields reached after work-up in experiments carried out in the presence of 10% DMSO were comparable to the results of Example 1. Enantiomeric purity was high for the **(*R*)-DCPI** enantiomer.

**EXAMPLE 3: Effect on the absence of glucose as co-substrate**

Setting **DCPE**-biomass ratio to 28 mg/g, the influence of glucose as co-substrate was studied in the presence and absence of DMSO as a co-solvent (Table 3).

A reaction flask with 100 mg of **DCPE** was charged with 100 mM sodium phosphate buffer and a volume of resting cells of *Rhodococcus ruber* to achieve a cell concentration of 90 g/l (wet cell basis; corresponds to a biomass relationship of 28 mg **DCPE**/g cells) in a final volume of 40 ml. The reaction was conducted on an orbital shaker (200 rpm) at 30°C for three days. The results are shown in Table 3.

Table 3

| ***Biomass Relationship (mg DCPE/g cells)*** | ***DMSO* (%)** | ***Conversion* (%)** | ***Yield* (%)** | ***Enantiomeric Purity (% R)*** |
|---|---|---|---|---|
| 28 | 0 | 43 | 35 | > 99 |
| 28 | 10 | 45 | 35 | > 99 |

The absence of co-substrate (glucose as reducing power) affected drastically the conversion resultants, reducing yields by half in comparison to Examples 1 and 2. Enantiomeric purity was high for the **(*R*)-DCPI** enantiomer.

**EXAMPLE 4: Co-solvent concentration study**

Reactions were performed in the optimal conditions of cell concentration and in the presence of glucose as co-substrate. Two solvents were tested in the range between 0 - 10% (DMSO and isopropanol).

A reaction flask with 25 mg of **DCPE**, a volume of co-solvent (DMSO, isopropanol) and 40 mg of glucose (as appropriate) was charged with 100 mM sodium phosphate buffer and a volume of resting cells of *Rhodococcus ruber* to achieve a cell concentration of 90 g/l (wet cell basis; corresponds to a biomass relationship of 28 mg **DCPE**/g cells) in a final volume of 10 ml. The reaction was conducted on an orbital shaker (200 rpm) at 30°C for 26 hours. After completion of the reaction, the pH of the suspension was shift to a pH of 12 and product was extracted with ethyl acetate (3x20 ml). Separation between phases is achieved by centrifugation (10000 rpm, 15 min). Solvent was evaporated to achieve yellow - brown oil. DMSO traces were removed by co-distillation with water in a rotary evaporator. The next steps followed the general procedure described above. The results are summarized in Table 4.

Table 4

| ***Co-solvent*** | ***[Co-solvent]*** **(%)** | ***Co-substrate*** | ***Conversion*** **(%)** | ***EP*** **(%** ***R)*** |
|---|---|---|---|---|
| isopropanol | 10 | glucose | 24 | > 99 |
| isopropanol | 5 | glucose | 59 | 98 |
| isopropanol | 0 | glucose | 96 | 99 |
| isopropanol | 10 | - | 12 | > 99 |
| isopropanol | 5 | - | 43 | 99 |
| DMSO | 10 | glucose | 89 | 99 |
| DMSO | 5 | glucose | 92 | > 99 |
| DMSO | 0 | glucose | 98 | > 99 |
| ***(*)*****:** Enantiomeric Purity | | | | |

The presence of isopropanol as co-solvent effected drastically the yield of conversion for the reaction of interest. Moreover, when isopropanol was used as co-substrate (absence of glucose) poor conversion was reached. Reactions carried out with 0 - 10 % DMSO achieved excellent conversion yields.

**EXAMPLE 5: Reaction temperature set-up**

According to the optimal conditions found in previous examples, reactions were conducted in the range of temperatures from 20°C to 50°C.

A reaction flask with 25 mg of **DCPE** and 40 mg of glucose was charged with 100 mM sodium phosphate buffer and a volume of resting cells of *Rhodococcus ruber* to achieve a cell concentration of 90 g/l (wet cell basis; corresponds to a biomass relationship of 28 mg **DCPE**/g cells) in a final volume of 10 ml. The reaction was conducted on an orbital shaker (200 rpm) at 30°C for three days. 0.5 ml samples were withdrawn at 24 and 72 hours reaction times and diluted with 1 ml of methanol. After blending the sample, *Rhodococcus ruber* resting cells were separated by centrifugation. A sample of the free-cell supernatant was diluted with methanol and analyzed by HPLC following the methodology reported above.

Quantitative conversion was achieved when the reaction was conducted at 30°C. Temperatures below 30°C slowed down the reaction, whereas poor conversions were achieved for reactions conducted at 37°C and 50°C.

**EXAMPLE 6: Reaction time set-up**

In order to study the progress of the reaction, different samples were withdrawn at different reaction time (from 1 to 92 hours).

A reaction flask with 25 mg of **DCPE** and 40 mg of glucose was charged with 100 mM sodium phosphate buffer and a volume of resting cells of *Rhodococcus ruber* to achieve a cell concentration of 90 g/l (wet cell basis; corresponds to a biomass relationship of 28 mg **DCPE**/g cells) in a final volume of 10 ml. The reaction was conducted on an orbital shaker (200 rpm) at 30°C for four days. 0.5 ml samples were withdrawn at different reaction times and diluted with 1 ml of methanol. After blending the sample, *Rhodococcus ruber* resting cells were separated by centrifugation. A sample of the free-cell supernatant was diluted with methanol and analyzed by HPLC according to the methodology reported above.

Results are shown in Table 5.

Table 5

| ***Time (hours)*** | ***Conversion*** **(%)** | ***EP*** **(%** ***R)*** |
|---|---|---|
| 0 | 1 | > 99 |
| 1.75 | 12 | > 99 |
| 3.25 | 16 | > 99 |
| 20 | 35 | 99 |
| 24 | 42 | > 99 |

(continued)

| *Time (hours)* | *Conversion* (%) | *EP* (% *R*) |
|---|---|---|
| 44 | 99 | > 99 |
| 92 | 100 | > 99 |
| EP: Enantiomeric Purity | | |

Complete conversion was achieved in less than 2 days reaction for the reaction conditions used in the example.

**EXAMPLE 7: Reaction pH set-up**

According to the optimal conditions found in previous examples, reactions were conducted in the range of pH from 5 to 9.

A reaction flask with 25 mg of **DCPE** and 40 mg of glucose was charged with 100 mM sodium phosphate buffer and a volume of resting cells of *Rhodococcus ruber* to achieve a cell concentration of 90 g/l (wet cell basis; corresponds to a biomass relationship of 28 mg **DCPE**/g cells) in a final volume of 10 ml. The reaction was conducted on an orbital shaker (180 rpm) at 30°C for two days. 0.2 ml samples were withdrawn at 18 and 40 hours reaction times and diluted with 1.8 ml of methanol. After blending the sample, *Rhodococcus ruber* resting cells were separated by centrifugation. A sample of the free-cell supernatant was diluted with methanol and analyzed by HPLC following the methodology reported above.

The results show (Figure 1) that quantitative conversion was achieved when the reaction was in the range of pH between 7 and 8, with faster conversion at pH 8. pHs below 7 slowed down the reaction, and poor conversions were achieved after 40 hours of reaction. When reaction was conducted at pH 9, 80% conversion was achieved after 40 hours.

**EXAMPLE 8: Semipreparative biocatalytic reduction of DCPE**

Setting the optimal conditions for all the parameters studied in the previous examples, a preparative reaction starting with 400 mg of **DCPE** and 700 mg of glucose was performed in 130 ml of 90 g/L *Rhodococcus ruber* resting cells suspension in 100 mM phosphate buffer pH 7.9. The reaction was conducted on an orbital shaker (180 rpm) at 30°C for three days. 0.5 ml samples were withdrawn at different reaction times and diluted with 1 ml of methanol. After blending the sample, *Rhodococcus ruber* resting cells were separated by centrifugation. A sample of the free-cell supernatant was diluted with methanol and analyzed by HPLC according to the methodology reported above.

After completion of the reaction, the pH of the suspension was shift to a pH of 12 and product was extracted with ethyl acetate (4x50 ml). Separation between phases is achieved by centrifugation (10000 rpm, 12 min). Solvent was evaporated to achieve yellow - brown oil which was dissolved in 1M hydrochloric acid solution. Colored impurities were extracted with ethyl acetate (3x10 ml). Then, solution was adjusted to pH > 12 with sodium hydroxide and **(*R*)-DCPI** was extracted with ethyl acetate (3x10 ml). Organic phase was dried with sodium sulfate and solvent was removed to give a light yellow glassy mass. The mass was recrystallised from hexane (25 ml) to give the product **(*R*)-DCPI** as a light yellow solid (yields are summarized in Table 6).

Table 6

| *Starting material* | *Product reaction* | *Conversion* (%) | *Yield* (%) | *EP* (% R) |
|---|---|---|---|---|
| **DCPE** | **(R)-DCPI** | HPLC | After work-up | HPLC |
| 400 mg (1.56 mmol) | 319 mg (1.25 mmol) | > 99% | 80% | 99% |
| EP: Enantiomeric Purity | | | | |

After work-up, spectroscopic characterization of the product ***(R)*-DCPI** was done ($^{1}$H-NMR, $^{13}$C-NMR, FT-IR and specific rotation).

$^{1}$H-NMR (400 MHz, $CDCl_3$) δ 3.84 (dd, J = 8.4 and 14.4 Hz, 1 H), 4.18 (dd, J = 2 and 14 Hz, 1 H), 5.20 (dd, J = 2.4 and 8.4, 1 H), 6.78 (s, 1 H), 6.88 (s, 1 H), 7.28 (dd, J = 2 and 8.4 Hz, 1 H), 7.33 (s, 1 H), 7.38 (d, J = 2, 1 H), 7.58 (d, J = 8.4 Hz, 1 H).

$^{13}$C-NMR (100 MHz, $CDCl_3$) δ 53.5, 69.4, 119.5, 127.6, 128.2, 128.7, 129.0, 131.9, 134.1, 137.5 FT-IR ($cm^{-1}$) 3420 st(O-H), 3114 st(C-H, heterocycle), 1515 st (C-N), 1077 st(C-Cl, aromatic)

$[\alpha]^{25}_D$ = - 95.54 (c = 1.0003, MeOH, ee 97%); standard **(*R*)-DCPI** $[\alpha]^{25}_D$ = -98.94 (c = 1.0097, MeOH).

Quantitative conversion was achieved starting from 400 mg of **DCPE** and following the optimal conditions found in previous examples (90 g/l resting cells in phosphate buffer pH 7.9, 30°C, biomass ratio 34 mg/g cells and 3 days). After work-up, 319 mg of **(*R*)-DCPI** were obtained (99% conversion, 80% yield). Product was identified as **(*R*)-DCPI** according to spectroscopic data as compared to an independent sample of **(*R*)-DCPI** standard.

The following examples show the study of the biocatalytic reduction of **DCPE** with other microorganisms under screening conditions.

**EXAMPLE 9: Biocatalytic reduction of DCPE by *Pseudomonas fluorescens***

In an Eppendorf tube with 1 ml of *Pseudomonas fluorescens* resting cells prepared from a 3 ml culture were added DCPE (20 mM) and glucose (22 mM). The experimental conditions and results are summarized in Table 7.

Table 7

| ***Starting material,* DCPE concentration** | **Glucose concentration** | ***Temperature, rpm, time*** | ***Conversion* (%) HPLC** | ***EP* (% R) HPLC** |
|---|---|---|---|---|
| **20 mM** | **22 mM** | 28°C, 180 rpm, 48h | 4% | 99% |
| EP: Enantiomeric Purity | | | | |

**EXAMPLE 10: Biocatalytic reduction of DCPE by *Saccharomyces cerevisiae***

The reaction was carried out by following the procedure described in Example *9. Saccharomyces cerevisiae* resting cells were prepared from a 3 ml culture. The experimental conditions and results are summarized in Table 8.

Table 8

| ***Starting material,* DCPE concentration** | **Glucose concentration** | ***Temperature, rpm, time*** | ***Conversion* (%) HPLC** | ***EP* (% R) HPLC** |
|---|---|---|---|---|
| **31 mM** | **28 mM** | 35°C, 300 rpm, 24h | 12% | 96% |
| EP: Enantiomeric Purity | | | | |

**EXAMPLE 11: Biocatalytic reduction of DCPE by *Schizochytrium limacinum***

The reaction was carried out by following the procedure described in Exemple *9.* ***Schizochytrium limacinum*** resting cells were prepared from a 50 ml culture. The experimental conditions and results are summarized in Table 9.

Table 9

| ***Starting material,* DCPE concentration** | **Glucose concentration** | ***Temperature, rpm, time*** | ***Conversion* (%) HPLC** | ***EP* (% *R*) HPLC** |
|---|---|---|---|---|
| **10 mM** | **11 mM** | 30°C, 200 rpm, 16h | 50% | > 99% |
| EP: Enantiomeric Purity | | | | |

**EXAMPLE 12: Biocatalytic reduction of DCPE by *Lactobacillus kefiri***

The reaction was carried out by following the procedure described in Example *9.* ***Lactobacillus kefiri*** resting cells were prepared from a 50 ml culture. The experimental conditions and results are summarized in Table 10.

Table 10

| ***Starting material,* DCPE concentration** | ***Glucose* concentration** | ***Temperature, rpm, time*** | ***Conversion* (%) HPLC** | ***EP* (% *R*) HPLC** |
|---|---|---|---|---|
| **20 mM** | **44 mM** | 30°C, 150 rpm, 66h | 16% | > 99% |
| EP: Enantiomeric Purity | | | | |

**Claims**

1. A process for the manufacture of the compound of formula (**I**)

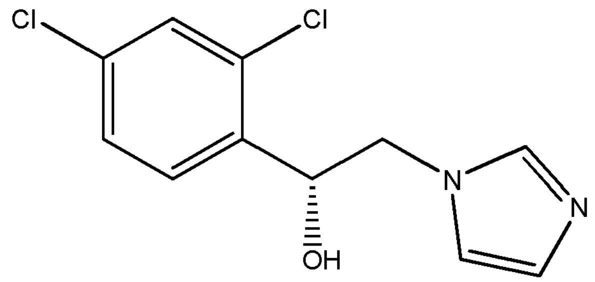

**(*R*)-DCPI (I)**

which comprises

i) bringing into contact a compound of formula (**II**)

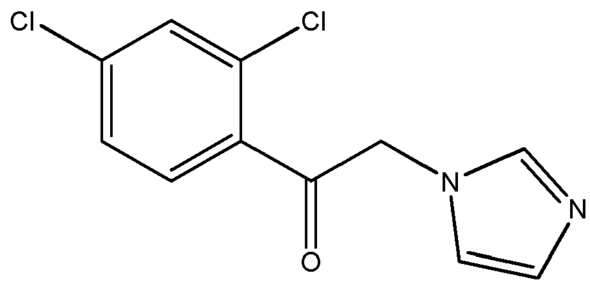

**DCPE (II)**

with a microorganism, wherein said microorganism has ketone asymmetric reduction activity; and
ii) recovering the compound of formula (**I**) so produced.

2. The process according to claim 1, wherein said microorganism is selected from the group consisting of bacteria, fungi, microalgae, protist, and archaea.

3. The process according to claim 2, wherein said microorganism is a bacterial cell.

4. The process according to any one of claims 1 to 3, wherein the microorganism is in the form of a culture broth, separated cells from the culture broth, treated preparations of cells or immobilized forms thereof.

**5.** The process according to any one of claims 1 to 4, wherein the microorganism is selected from the group consisting of a microorganism of the genus *Saccharomyces, Schizochytrium, Lactobacillus, Rhodococcus,* and *Pseudomonas.*

**6.** The process according to claim 5, wherein the microorganism is selected from the group consisting of *Saccharomyces cerevisiae, Schizochytrium limacinum, Lactobacillus kefiri, Rhodococcus ruber,* and *Pseudomonas fluorescens.*

**7.** The process according to claim 6, wherein the microorganism is *Rhodococcus ruber.*

**8.** A process for the manufacture of the (*R*)-enantiomer of Sertaconazole (Arasertaconazole)

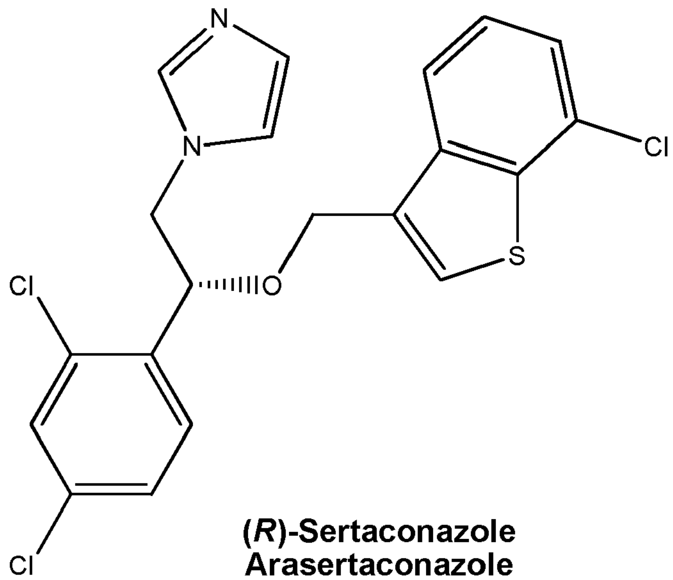

**(*R*)-Sertaconazole**
**Arasertaconazole**

which comprises:

i) bringing into contact a compound of formula (**II**)

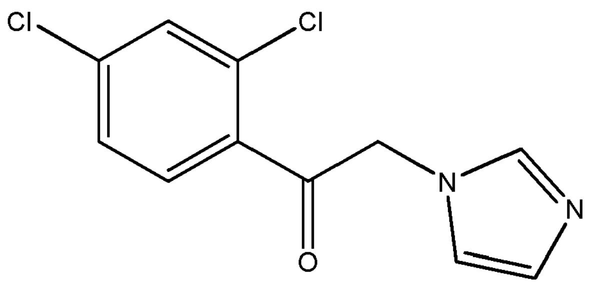

**DCPE** (**II**)

with a microorganism, wherein said microorganism has ketone asymmetric reduction activity; and
(ii) O-alkylating the compound of formula **(*R*)-DCPI** (**I**) obtained according to step i)

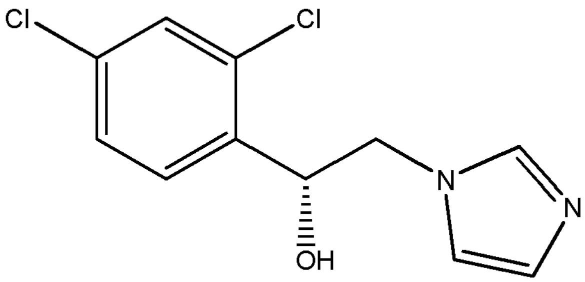

**(*R*)-DCPI** (**I**)

with a compound of formula (**III**)

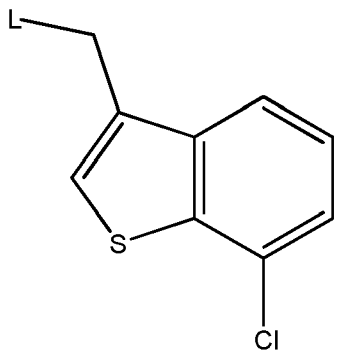

(**III**)

wherein L is a leaving group selected from the group consisting of chloride, bromide, iodide, tosylate, mesyl, and trifluoromethylsulfonyl.

**9.** The process according to claim 8, further comprising a step iii) of reacting the compound obtained according to step ii) with an acid compound to form a pharmaceutically acceptable salt thereof.

**10.** The process according to any one of claims 8 or 9, wherein said microorganism is selected from the group consisting of bacteria, fungi, microalgae, protist, and archaea.

**11.** The process according to claim 10, wherein said microorganism is a bacterial cell.

**12.** The process according to any one of claims 8 to 11, wherein the microorganism is in the form of a culture broth, separated cells from the culture broth, treated preparations of cells or immobilized forms thereof.

**13.** The process according to any one of claims 8 to 12, wherein the microorganism is selected from the group consisting of a microorganism of the genus *Saccharomyces, Schizochytrium, Lactobacillus, Rhodococcus,* and *Pseudomonas.*

**14.** The process according to claim 13, wherein the microorganism is selected from the group consisting of *Saccharomyces cerevisiae, Schizochytrium limacinum, Lactobacillus kefiri, Rhodococcus ruber,* and *Pseudomonas fluorescens.*

**15.** The process according to claim 14, wherein the microorganism is *Rhodococcus ruber.*

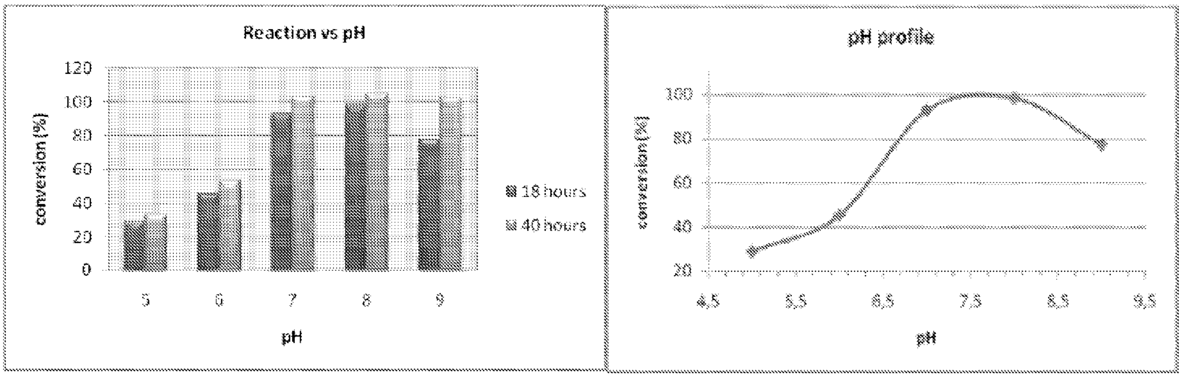
Reaction vs pH
conversion (%)
120
100
80
60
40
20
0
5
6
7
8
9
pH
18 hours
40 hours
pH profile
conversion (%)
100
80
60
40
20
4,5
5,5
6,5
7,5
8,5
9,5
pH

*FIGURE 1*

Europäisches Patentamt
European Patent Office
Office européen des brevets

# EUROPEAN SEARCH REPORT

Application Number
EP 11 17 5965

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | MANGAS-SÁNCHEZ ET AL: "Asymmetric chemoenzymatic synthesis of miconazole and econazole enantiomers. The importance of chirality in their biological evaluation", THE JOURNAL OF ORGANIC CHEMISTRY, vol. 76, 8 March 2011 (2011-03-08), pages 2115-2122, XP002665814, * See page 2117 (Scheme 1/ reaction 3 -> 4); early online publication * ----- | 1-15 | INV. C12P17/10 |
| A | GARCÍA-URDIALES ET AL: "Update 1 of: Enantioselective enzymatic desymmetrizations in organic synthesis", CHEMICAL REVIEWS, vol. 111, 7 March 2011 (2011-03-07), pages PR110-PR180, XP002665852, * See pages PR131 (right column / Rhodococcus), PR 132 (Scheme 38), and PR 135 (Scheme 40); early online publication * ----- | 1-15 | |
| | | | TECHNICAL FIELDS SEARCHED (IPC) |
| A | STAMPFER ET AL: "Biocatalytic asymmetric hydrogen transfer employing Rhodococcus ruber DSM 44541", THE JOURNAL OF ORGANIC CHEMISTRY, vol. 68, 2003, pages 402-406, XP002269816, * See page 403 (Figure 1 and Table 1) * ----- | 1-15 | C12P |
| A | EP 2 348 120 A1 (UNIVERSITÄT WIEN / TECHNISCHE UNIVERSITÄT GRAZ) 27 July 2011 (2011-07-27) * See pages 2-3 (Objects and summary of the invention) * ----- | 1-15 | |
| | -/-- | | |

The present search report has been drawn up for all claims

2

| Place of search | Date of completion of the search | Examiner |
|---|---|---|
| Berlin | 15 December 2011 | Korsner, Sven-Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 17 5965

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 0 493 617 A1 (KANEGAFUCHI KAGAKU KOGYO KABUSHIKI KAISHA) 8 July 1992 (1992-07-08) * See page 2 (Disclosure of the invention) * ----- | 1-15 | |
| A,D | CN 1 765 887 A (ZHEJIANG UNIVERSITY) 3 May 2006 (2006-05-03) * See the Figures * ----- | 1-15 | |
| | | | TECHNICAL FIELDS SEARCHED (IPC) |

The present search report has been drawn up for all claims

2

| Place of search | Date of completion of the search | Examiner |
|---|---|---|
| Berlin | 15 December 2011 | Korsner, Sven-Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 17 5965

This annex lists the patent family members relating to the patent documents cited in the above-mentioned European search report.
The members are as contained in the European Patent Office EDP file on
The European Patent Office is in no way liable for these particulars which are merely given for the purpose of information.

15-12-2011

| Patent document cited in search report | Publication date | Patent family member(s) | Publication date |
|---|---|---|---|
| EP 2348120 A1 | 27-07-2011 | NONE | |
| EP 0493617 A1 | 08-07-1992 | DE 69131685 D1 | 11-11-1999 |
| | | DE 69131685 T2 | 27-04-2000 |
| | | EP 0493617 A1 | 08-07-1992 |
| | | US 5266485 A | 30-11-1993 |
| | | WO 9201804 A1 | 06-02-1992 |
| CN 1765887 A | 03-05-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 1244530 A
- WO 03068770 A
- CN 1765887


### Non-patent literature cited in the description


- **LÄMMERHOFER M. ; LINDER W.** *Chirality,* 1994, vol. 6, 261-269
- **MANGAS-SANCHEZ, J. et al.** *Journal of Organic Chemistry,* 2011, vol. 76 (7), 2115-2122